# EUROPEAN PATENT SPECIFICATION

(11) **EP 1 285 667 B1**
(45) Date of publication and mention of the grant of the patent: **14.06.2006**
(21) Application number: 02023125.4
(22) Date of filing: 18.11.1997
(51) Int. Cl.: A61K 47/10, A61K 47/12, A61K 31/165

(54) **Pharmaceutical injectable solution of paracetamol and combinations of paracetamol with other active substances**
Pharmazeutische injizierbare Lösungen, die Paracetamol und Kombinationen aus Paracetamol mit anderen Aktivsubstanzen enthalten
Solutions pharmaceutiques injectables contenant du paracétamol et des associations de paracétamol avec d' autres substances actives

(43) Date of publication of application: 26.02.2003
(62) Divisional of application: 97600009.1
(73) Proprietor: Uni-Pharma Kleon Tsetis Pharmaceutical Laboratories S.A., 145 64 Kifissia (GR)
(72) Inventor: Tsetis, Julia, 145 61 Kifissia (GR)

(56) References cited:
- GR-B- 1 001 523
- GR-B- 1 002 731
- US-A- 5 296 241

## Description

This invention refers to the pharmacotechnical field and has to do with pharmaceutical solutions of Hydroxy-acetanilide (paracetamol) or/and its combination with other active substances suitable from the pharmacotechnical and therapeutical point of view, for parenteral use.

Paracetamol is considered to be the main active metabolite of phenacetin and acetanidile having analgesic and antipyretic properties. Paracetamol has equivalent analgesic and antipyretic action to that of aspirin whilst it expresses weak anti-inflammatory action therefore its use in inflammatory rheumatic diseases is limited.

The mechanism of its analgesic action is still unclarified. It is believed that it mainly acts by inhibiting prostaglandins biosynthesis and to a lesser extent by peripherically inhibiting algogenic stimulus origin. The peripheral action is due also to inhibition of proglandins biosynthesis or to inhibition or to other endogenous substances action that sensitize pain's receptors after mechanic or chemical stimulation.

As far as its antipyretic action is concerned, Paracetamol induces temperature fall to feverish but not to normal subjects. It is believed that the antipyretic effect of Paracetamol is due to central action on the temperature controlled centre of hypothalamus resulting in peripheral vasodilation leading to skin peripheral blood flow increase, perspiration and temperature loss.

This peripheral action of Paracetamol is due also to prostaglandins biosynthesis inhibition into hypothalamus.

Paracetamol administered in recommended dosage does not exert any effect of the cardiovascular and respiratory system nor provokes acid-base balance disorders.
Several studies have confirmed the effectiveness and safety of Paracetamol's parenteral administration.
Paracetamol is well absorbed when intramuscularly administered and its blood level is similar to that obtained after its oral administration.

The absorption rate is slower of that obtained when Paracetamol is orally administered, resulting in desirable blood levels for more prolonged time.

There is also another advantage of injectable Paracetamol since the 20% loss of the drug that is observed after oral administration doesn't exist (Macheras et aL 1989, Pharmaceutical Codex 1994).

Paracetamol is metabolized by the microsomal enzymes of the liver and 95% of it is excreted through urines as conjugated derivatives of sulfuric (35%) and glucouronic acids (60%) whilst only 2% is excreted unchangeable (Gillette 1981, Clissold 1986, Remington 1990, Insel 1992, AMA-DE 1994).

Also a small part of Paracetamol, approx. 3%, is oxidized by the liver cytochrome P-450 to a toxic intermediate metabolite that is connected to the liver deposit of glutathione, producing finally a non-toxic combination, which is excreted conjugated with cysteine and mercapturic acid (Mitchell et al 1982, Jackson et at. 1984, Remington 1990, Insel 1992).

Fifteen years ago, combinations of Paracetamol plus other active substances as Le. analgesics (aspirin, codeine), spasmolytics (hyoscine-N-butyl-bromide, mebeverine etc), or substances antidote for Paracetamol's toxicity were administered orally or rectally but not parenterally (intramuscularly or intravenously).

Therefore, preparation of parenteral solutions of Paracetamol and combinations of it with other substances as above mentioned, are considered to be indispensable for use in the modern therapeutics for a quicker and greater therapeutical effect

Whilst Paracetamol is soluble in many organic solvents, however solutions of Paracetamol with such solvents are unfit for therapeutical use, because of the produced toxicity when parenterally administered (intramuscularly or intravenously) and because of the present technical problems as i.e. chemical instability leading to precipitates, low fluidity etc.

As above, the preparations of injectable solutions of Paracetamol and combinations of Paracetamol with other active substances require the choice of the suitable solvent or combination of solvents, comprising also water, reciprocating to certain requirements of suitability as : to be pharmacologically inactive, to not form complexes with the active substance, to be blood conventional, free of sensitization or irritating activity, chemically stable, clear and not influenced by pH declinations.

But it is important the selected solvents to not interfere with Paracetamols' or other's substances therapeutical properties.
From the pharmacotechnical point of view, the selected solvent or solvents system must have the full ability of mixing with water not only because this way it or they will facilitate the manufacturing process but will also reduce the manufacturing cost.

Furthermore, this property of fully mixing with water makes depended to a great extend the pharmacokinetic and bio-availability of the preparation and the local tolerance in the site of injection as well.

Gr 1002731 and Gr 1001523 disclose injectable solutions of paracetamol comprising a solvent system of glycerol formal, ethanol and water.

### The Invention

According to the present invention chemically stable solutions of Paracetamol are obtained, suitable for parenteral administration as well as solutions of Paracetamol with other active substances as i.e. with Codeine for a preparation with increased analgesic effect or with Hyoscine-N-Butylbromide for a preparation having spasmo-analgetic effect.

Since Paracetamol is not soluble in the water, efforts made for its dissolution into organic-solvents or mixtures of them, suitable for parenteral use.

Among the tested solvents proved suitable Ethanol, Benzylic Alcohol and GLYCEROL FORMAL Ethanol and Benzylic Alcohol are already used as solvents for parenteral administration of drugs, whilst GLYCEROL FORMAL (an almost atoxic substance having LD₅₀ to rats 3,5mg/kg body weight, when is given intravenously), having the property to be mixed in whichever rate with water and/or with Ethanol is the first time that has been applied in the preparation of parenteral use solution.

Our experiments for the discovery of proper solvents for Paracetamol's solubilization, have shown that there are at least two (2) systems in which other active substances that may be combined with Paracetamol for therapeutical purposes are also soluble.

### 1. Mixture of solvents consisting of Ethanol - Glycerol Formal Water in the ratio of 10:50:40 respectively.

To this mixture of solvents the following organic matters are added : Lidocaine HCl (local anaesthetic), Disodium Edetate, Sodium Metabisulphite (antioxidant agent) and Disodium Phosphate (buffer controlling pH to 5,5-5,6)

The solution resulting from the mixing of these matters with the mixture of solvents, is a dear and stable solution and constitutes the "basic" solution of Paracetamol for parenteral admini-stration which afterwards will be used as it is for the preparation of parenteral solution of Paracetamol's combinations with other active substances as i.e. Hyoscine-N-Butylbromide, Codeine phosphate etc

### "Basic" solutions: Composition of 4 ml amp.

| | |
|---|---|
| PARACETAMOL | 15,00 mg |
| LIDOCAM HCl | 0,50 mg |
| DISODIUM EDETATE | 0,01 mg |
| DISODIUM DIBASIC PHOSPHATE | q.s. |
| SODIUM METABISULFITE | 0,05 mg |
| GLYCEROL FORMAL | 37,50 ml |
| ETHANOL | 10,00 ml |
| YΔΩP (γlα ενέσεlζ) q.s. | 100,00 ml |

1. It has been proved that in the "basic" solution of Paracetamol for parenteral administration, Hyoscine-N-Butylbromide is soluble and that the so occurred solution is clear, stable and proper for parenteral (intramuscular) use combining the analgetic action of Paracetamol with the spasmolytic effect of Hyoscine-N-Butylbromide for the treatment of painful spastic-conditions of the splachnic organs.

### Composition of a 4 ml ampoule

| | |
|---|---|
| PARACETAMOL | 600,0 mg |
| HYOSCINE-N-BUTYLBROMIDE | 20,0 mg |
| LIDOCAINE HCI | 20,0 mg |
| DISODIUM EDETATE | 4,0 mg |
| DISODIUM DIBASIC PHOSPHATE | q.s |
| NIPAGIN A | 1,8 mg |
| NIPASOL M | 0,2 mg |
| GLYCEROL FORMAL | 1,5 mg |
| ETHANOL | 4,0 mg |
| WATER | q.s. |

### ITEMS RELATED TO THE INVENTION

1. Solution suitable for parenteral administration of matters not soluble in water consisting of 1) ETHANOL, GLYCEROL FORMAL and Water and 2) BENZYLIC ALCOHOL GLYCEROL FORMAL and Water.
2. Method for the preparation of injectable solution of Paracetamol into aqueous organic solution where solvents of the solution are ETHANOL, GLYCEROL FORMAL and Water, in the ratio 10:50:40 by volume.
3. The solution of item 2 comprises as excipients LIDOCAINE HCI (local anaesthetic) 5mg/ml, DISODIUM EDETATE 0,10mg/ml, DISODIUM DIBASIC PHOSPHATE (pH buffer) q.s. for pH 5,5-5,6, SODIUM METABISULFITE 0,5mg/ml.
4. Process for the preparation of injectable solution of Paracetamol into aqueous organic solution, where solvents consisting the solution are BENZYLIC ALCOHOL, GLYCEROL FORMAL and Water, in a ratio of 10:50:40 by volume.
5. Process for the preparation of injectable solution of Paracetamol and HYOSCINE-N-BUTYLBROMIDE based on the solution of item 2 and the excipients of item 3, after the elimination of SODIUM METABISULFITE and addition of NIPAGIN A and NIPASOL M.
6. Process for preparation of injectable solution of Paracetamol and HYOSCINE-N-BUTYLBROMIDE based on the solution of item 4 and the excipients of item 5.
7. Process for the preparation of Injectable combination solution of Paracetamol plus CODEINE PHOSPHATE according to items 1, 3 and 5.

### Literature

1. AMA-DE (1994): Acetaminophen. American Medical Association and American Society for Clinical Pharmacology and Therapeutics. American Medical Association Chicago p. 123-124.
2. Ayre-Smith G. (1976): Hyoscine-N-butylbromide (Buscopan) as a duodenal relaxant in tubeless duodenography; Acta Radiol. Diagnosis 17, 701-713.
3. Bouδoúρηç K. MΠαvάβα Kλ., Koúykα E., Kai σuv. (1988): Avαδρoµikη αvolktη µελέTη αvαληTlkηç δράσεωç εvέσlµηç αkεTαµlvoϕαiηç σTα ρεuµαTlkά voσήµαrα; ┌αληvóç, 30, 497-502.
4. Gillette J.R. (1981): An intergrated approach to the study of chemically reactive metabolites of acetaminophen; Arch. Intern. Med., 141, 375-379.
5. Goiz Duran L, Landa J., Martinez J.S. (1988): Empieo dei espasnioittico-analgesico IK-19 en espasmos viscerales de diversa efiologia; Invest. Med. lnter., 15, 26-28.
6. Graubner W. (1966): Hyoscine butylbromide in man; Lancet, 2, 700.
7. Herxheimer A. Haefell L. (1966): Human pharmacology of hyoscine butylbromide; Lancet, 2, 418-421.
8. Insel P.A. (1992): Analgesic-antipyretics and antiiflammatory agents; Drugs employed in the treatment of rheumatoid asthritis and gout in Goodman and Gilman's. The pharmacologital Besis of Therapeutics, 8th Edition, McGraw-Hill Inc. New York p. 638-641, 656-659.
9. Jachson C.H., Mac Donald N.C., Comett J.W.D. (1984): Acetaminophen: a practical pharmacologic overview. Can.Med. Assoc.J., 131, 25-32.
10. Jaeger A., Flesch F, Kopferschmitt J., et al (1987): Intoxication algue par le paracetamol; Revue du Praticien 37, 2881-2886.
11. Janes J., Routledge PA (1992): Recent developments in the management of paracetamol (acetaminophen) poisoning; Drug Safety 7, 170-177.
12. Landa Palow M.J., Martinez J.S. (1988): Valoracion clinica de la combinacion espasmolttica-analgestca del IK-19 en suposttorios; Invest. Med Intern, 15, 45-47.
13. Macheras P., Partssi-Poulou M, Poulos L (1989): Pharmacokinetics of acetaminophen after intramuscular administration; Biopharm. Drug Dispos., 10, 101-105.
14. Mitchell J.R., Hughes H, Lauterburg B.H., Smith C.V. (1982): Chemical nature of reactive intermediates as determinant of toxicologic responses; Drug Metab. Rev., 13, 539-553.
15. Miyoshi A, Suyama I., Kawamura L (1977): A double-blind comparative study of inhibitory effect of intraduodenally administered hyoscine N-butyl bromide on human duodenal motility; Jap. J. Int. Med. Res., 4, 223-232.
16. Pharmaceutical Codex (1994): The Pharmaceutical Codex, 12th Ed., Ed Walter Lund, London, The Pharmaceutical Press, p. 987-992.
17. Remington (1990): Remington's Pharmaceutical Sciences, 18th Ed., Mack Publ. Easton, p. 907-909, 1109-1110.
18. Rumack B.H., Brent J. (1992): Acetaminophen poisoning; in Hall JB. et aL Principles of Critical Care, McGraw-Hill, New York, p. 2136-2140.
19. Schmid E., Bleichert A., Uberia K., et al. (1968b): Investigations into the testing of oral antispasmodics as demonstrated by the effect of hyoscine N-butylbromide (Buscopan) on gastric motility; Drugs Made in Germany, 11, 153-163.
20. Vasquez G.V. (1979): Usos del compuesto IK-19 en cuadros espasmo-dolorosos del tracto genital; Revista Colombtana de Obstetricia y Ginecologia, 30, 169-173.

## Claims

1. Solutions suitable for parenteral administration comprising
a) the actives paracetamol and hyoscine-N-butylbromide,
b) the solvents ethanol, glycerol formal and water at a volume ratio ethanol:glycerol formakwater 10:50:40, as well as
c) 5mg/ml lidocaine HCI, 0.1mg/ml disodium edetate, q.s. disodium dibasic phosphate for a pH 5.5-5.6, nipagin A and nipasol M, eliminating sodium metabtsulfite.

2. Solution according to claim 1, including further active substances such as codeine phosphate.

3. Solution suitable for 4ml ampoule comprising 600mg paracetamol, 20mg hyoscine-N-butylbromide, 20mg lidocaine HCl, 4mg disodium edetate, disodium dibasic phosphate q.s., 1.8mg nipagin A, 0.2mg nipasol M, 15mg glycerol formal, 4mg ethanol and q.s. water.

## Patentansprüche

1. Geeignete Lösungen für parenterale Darreichung enthaltend
a) die aktive Substanzen Parazetamol und Hyoscine-N-Butylbromid,
b) die Lösungsmittel Ethanol, Formal Glyzerol und Wasser in einem Volumenverhältnis Ethanol : Formal Glyzerol : Wasser von 10:50:40 als auch,
c) 5mg/ml Lidocain HCl, 0,1mg/ml Disodium EDTA, Disodium Dibasic Phosphat q.s. für pH 5,5-5,6, Nipagin A und Nipasol M, außer Sodium Mebabisulfit.

2. Lösung gemäß Anspruch 1, die weitere aktive Substanzen wie Codein Phosphat enthält.

3. Geeignete Lösung für eine 4ml Ampulle, die 600mg Parazetamol, 20mg Hyoscine-N-Butylbromid, 20mg Lidocain HCl, 4mg Disodium EDTA, Disodium Dibasic Phosphat q.s., 1,8mg Nipagin A, 0,2mg Nipasol M, 1,5mg Formal Glyzerol, 4mg Ethanol und Wasser q.s. enthält.

## Revendications

1. Solutions utilisées pour administration parentérale comprenant:
a) les principes actifs paracétamol et hyoscine-N-butylbromide,
b) les solvants éthanol, glycérol formal et de l'eau avec un rapport d'éthanol : glycérol formal : eau égal à 10 :50 :40, ainsi que
c) 5mg/ml de lidocaïne HC1, 0,1 mg/ml d'EDTA disodique, q.s.p phosphate dibasique de disodium pour un pH=5,5-5,6, nipagin A et nipasol M, en éliminant métabisulfite de sodium.

2. La solution selon la revendication 1 comprenant de principes actifs supplémentaires tels que le phosphate de codéine.

3. Solution convenable pour une ampoule de 4ml comprenant 600mg de paracétamol, 20mg de hyoscine-N-butylbromide, 20mg de lidocaïne HCl, 4mg d'EDTA disodique, du phosphate dibasique de disodium q.s., 1,8mg de nipagin A, 0,2mg de nipasol M, 1,5mg de glycérol formal, 4mg d'éthanol et de l'eau q.s.p.
